# FASCICULE DE BREVET EUROPEEN

(11) **EP 1 885 337 B1**
(45) Date de publication et mention de la délivrance du brevet: **01.08.2012**
(21) Numéro de dépôt: 06763147.3
(22) Date de dépôt: 15.05.2006
(51) Int. Cl.: A61K 9/18, A61P 35/00, A61K 9/14, A61K 39/00

(54) **MEDICAMENT NOTAMMENT ANTI-CANCEREUX, DESTINE A DES TRAITEMENTS PAR IMMUNOTHERAPIE, EN PARTICULIER AUTOLOGUE**
ARZNEIMITTEL, INSBESONDERE ANTIKREBSMITTEL, ZUR BEHANDLUNG UNTER VERWENDUNG VON IMMUNTHERAPIE, INSBESONDERE AUTOLOGER
MEDICAMENT, PARTICULARLY AN ANTI-CANCER MEDICAMENT, FOR TREATMENT USING IMMUNOTHERAPY, PARTICULARLY AUTOLOGOUS

(30) Priorité: 13.05.2005 FR 0551262
(43) Date de publication de la demande: 13.02.2008
(73) Titulaire: Urodelia, 31470 Saint Lys (FR); Ciocca, Daniel, 5500 Mendoza (AR)
(72) Inventeur: FRAYSSINET, Patrick, F-31470 Saint Lys (FR); ROUQUET, Nicole, F-31400 Toulouse (FR); CIOCCA, Daniel, 5500 Mendoza (AR)
(74) Mandataire: Cabinet Plasseraud
(86) Numéro de dépôt international: PCT/EP2006/062296
(87) Numéro de publication internationale: WO 2006/122914

(56) Documents cités:
- WO-A-98/35562
- WO-A-2004/060407
- DE-A1- 3 426 049
- FR-A- 2 543 439
- JP-A- 59 197 595
- US-A1- 2002 176 845
- US-A1- 2003 082 232
- US-B1- 6 541 000
- PAUL W ET AL: "Development of porous spherical hydroxyapatite granules: application towards protein delivery" JOURNAL OF MATERIALS SCIENCE. MATERIALS IN MEDICINE, CHAPMAN AND HALL, LONDON, GB, vol. 10, no. 7, 1999, pages 383-388, XP002142926 ISSN: 0957-4530

## Description

### Champ d'application :

Le domaine de l'invention est celui des traitements par immunothérapie, en particulier des traitements des cancers, des maladies infectieuses ou des maladies auto-immunes et des moyens qui y sont associés.

Plus précisément, l'invention a trait à des traitements autologues par immunostimulation. Plus précisément encore, l'invention concerne des moyens de lutte notamment contre le cancer, comprenant des vaccins anti-tumoraux obtenus à partir des antigènes tumoraux des patients.

La présente invention vise notamment un médicament, par exemple un vaccin antitumoral, à base de microparticules minérales (e.g. hydroxyapatite) ou polymériques, biocompatibles.

L'invention concerne également un procédé de préparation d'antigènes tumoraux destiné à les mettre sous une forme reconnaissable par le système immunitaire et plus particulièrement pour purifier les antigènes tumoraux d'un patient déterminé afin de réaliser une autovaccination générant une réponse immunitaire spécifique contre les cellules tumorales du patient.

### Etat de la technique :

Les formes de traitement contre le cancer par immunothérapie sont actuellement de trois types :
I.- *Administration de substances pharmacologiques qui augmentent la réponse immunitaire de manière non spécifique.* Il peut s'agir par exemple de l'administration d'interleukines ou d'interféron aux patients ayant une tumeur maligne rénale ou bien un mélanome. Une autre thérapie non spécifique est l'administration de BCG chez les patients ayant un cancer de la vessie. Ces thérapies ont un degré variable de toxicité et bien qu'elles puissent montrer une certaine efficacité, beaucoup de patients ainsi traités répondent faiblement Ces thérapies provoquent une amplification de nombreuses fonctions immunitaires.
II.- *Utilisation d'anticorps spécifiques contre les antigènes tumoraux présents à la surface des cellules tumorales.* Un des exemples est l'administration d'anticorps monoclonaux dirigés contre l'oncoprotéine HER-2/neu. Cette oncoprotéine est exprimée dans un faible pourcentage (20-30%) des patientes ayant un cancer du sein. L'anticorps monoclonal a été modifié par ingénierie génétique pour que seulement une petite partie de la molécule soit d'origine non humaine (la partie reconnaissant l'antigène est produite par le rat), le reste de la molécule est d'origine humaine. C'est en fait un anticorps hybride humanisé. Il a été fabriqué divers anticorps hybrides qui ont été évalués et certains sont utilisés cliniquement dans le traitement de divers types de tumeurs malignes. L'union des anticorps à leur antigène spécifique amène la cellule tumorale à ralentir sa prolifération et à la mort. Bien que ces traitements soient une avancée significative, leur efficacité est limitée en raison de la variabilité antigénique des cellules tumorales. Dans une tumeur solide, au moment du diagnostic, il y a des millions de cellules tumorales et parmi cette population, il existe certaines cellules exprimant les antigènes d'intérêt et d'autres non.
III.- *Stimulation des cellules T.* Cette forme de thérapie est en rapport avec l'objet de l'invention.

Le type III comprend l'immunothérapie adoptive, nommée ainsi car adoptée par le patient. Elle n'est pas innée, elle se développe loin du patient (*ex vivo*), avec le but d'augmenter le nombre de cellules T antitumorales. Ceci est obtenu en administrant au patient des cellules T cytotoxiques qui ont été générées et amplifiées *in vitro.*

Le type III englobe également l'immunothérapie active basée sur la stimulation des cellules T. Ceci se produit grâce à la mise en contact des antigènes tumoraux avec les macrophages ou des cellules apparentées (comme par exemple les cellules dendritiques de la peau : les cellules de Langerhans), qui présentent les antigènes afin que ces cellules dites APC (Antigen Presenting Cells : cellules présentatrices de l'antigène) stimulent les lymphocytes T. Cette stratégie peut se faire *in vivo* ou *in vitro.* Ces cellules APC initient, plus qu'elles ne maintiennent l'immunité antitumorale spécifique issue des lymphocytes T. L'immunothérapie active est l'objet de nombreuses recherches pour diverses tumeurs. Ces agents stimulant les lymphocytes T sont assimilables à des vaccins. Ainsi, il existe plusieurs stratégies A/, B/, C/, D/ pour générer des vaccins stimulant le système T sous forme active et spécifique :
A/ Stratégie d'immunothérapie reposant sur l'emploi de cellules dendritiques et autres cellules (in vitro). Lorsqu'elles sont en phase de croissance, elles sont mises en contact, en dehors de l'organisme, avec les antigènes tumoraux du patient (lysat tumoral autologue, cellules tumorales irradiées, peptides tumoraux spécifiques) et sont injectées intradermiquement au même patient (le reste se cryopréserve pour d'autres injections).
B/ Stratégie d'immunothérapie reposant sur l'emploi d'antigènes spécifiques et de molécules stimulantes. Les antigènes tumoraux spécifiques pouvant être utilisés dans des vaccins sont nombreux, par exemple des peptides produits de gènes mutés comme le ras, p53, VHL, qui sont associés à des adjuvants tels que le Montanide ISA 51. On peut également utiliser des peptides synthétiques ou recombinants solubles de fractions d'antigènes tumoraux spécifiques comme le MART-1 (mélanomes) ou le PSA (prostate).
C/ Stratégie d'immunothérapie reposant sur l'emploi des cellules tumorales ou d'un lysat de cellules tumorales. Les cellules tumorales utilisées dans cette stratégie peuvent être des cellules autologues ou allogéniques inactivées de diverses manières (radiation, congélation, chaleur, lumière ultraviolette), ou bien des lysats de cellules tumorales (les cellules sont homogénéisées).
D/ Stratégies d'immunothérapie reposant sur l'emploi de protéines du coup de chaleur comme adjuvant :

Dans ce cas, on extrait de la tumeur des protéines du coup de chaleur (HSPs) comme la gp 96, ou l'HSP 70. Ces protéines agissent comme chaperons moléculaires, et transportent des peptides (antigènes) spécifiques de la tumeur de chaque patient.

La capacité des HSPs de se lier à quasiment tout le protéome cellulaire fait de ces molécules une bonne option comme vaccination antitumorale pour trois raisons principales :
- Il n'est pas utile de connaître tout le protéome tumoral puisque la purification des seules HSPs permet d'isoler un grand nombre de protéines qui leurs sont liées
- Leur injection permet en une seule fois l'introduction dans l'organisme d'une grande quantité de protéines tumorales sous une forme reconnaissable par le système immunitaire.
- Les HSPs permettent le captage, le transit et le remodelage des protéines qui leur sont associées par les cellules présentatrices de l'antigène afin d'être présentées aux lymphocytes T.

Une des caractéristiques de l'utilisation de ces protéines comme vaccin antitumoral est la nécessité d'utiliser des HSPs provenant de la tumeur à éradiquer car elles constituent une empreinte moléculaire de celle-ci et diffèrent d'un patient et d'une tumeur à l'autre.

Ceci impose de purifier les protéines vaccinantes à partir de chaque tumeur contre laquelle elles doivent immuniser le patient. Le protocole de purification est long, difficilement industrialisable et sujet à de multiples contaminations par endotoxines. Il est classique de purifier les HSPs à partir d'un broyat tumoral qui est soumis à une série de centrifugation, précipitation, chromatographie sur Con A, analyse électrophorétique, et chromatographie sur Mono Q FPLC.

Les brevets US Nos 6,447,781, 6,436,404, 6,410,028, 6,383,494 et 6,030,618 décrivent des méthodes de purification de protéines HSP consistant à mettre en oeuvre des colonnes chromatographiques de Con A SEPHAROSE. Ces méthodes sont perfectibles.

On connaît par ailleurs des méthodes de purification faisant appel aux phosphates de calcium et en particulier à l'hydroxyapatite (HA). Les poudres d'hydroxyapatite sont utilisées depuis une trentaine d'années pour purifier diverses molécules biologiques comme des protéines, acides nucléiques, endotoxines et même virus. Les poudres sont utilisées comme lit fixe dans des colonnes de chromatographie à travers lesquelles est percolée la solution contenant la ou les molécules à purifier. Ces molécules se fixent sur la surface des particules de poudre, d'où elles sont désorbées à l'aide de concentrations diverses solutions salines tels que des tampons phosphates ou bien du chlorure de sodium, ce qui permet de séparer les différentes molécules de la solution par des gradients de tampons phosphate ou autres.

Les céramiques d'HA sous forme poreuses sont également utilisées depuis une vingtaine d'années comme substitut osseux. Elles sont intégrées dans le tissu osseux suivant une séquence d'événements biologiques se succédant et pouvant être énumérés comme il suit :
- Invasion des pores par des cellules circulantes
- Colonisation par un tissu conjonctif lâche.
- Formation de tissu ostéoide à la surface du matériau et minéralisation
- Remodelage de l'os formé et dégradation des céramiques.

La demande de brevet US 2003/0082232 décrit l'utilisation de support à base de phosphate de calcium et notamment d'hydroxyapatite comme adjuvant et vecteur pour les vaccins éventuellement en combinaison avec un agent actif, cet agent actif étant un composé ayant subi un traitement de purification. Parmi les agents actifs cités, on notera des protéines purifiées (par exemple : la cytokine (GM-CSF ou Granulocyte macrophage-colony stimulating factor), les toxines diphtérique et tétanique purifiées, l'hémocyanine Keyhole-limpet), de la poussière, le virus humain désactivé HIV-2, un plasmide exprimant une protéine de *Plasmodium yoelii* (PyCSP), une bactérie (*Bordetella pertussis*) ou encore un vaccin. L'adjonction d'agent(s) actif(s) à la préparation d'adjuvant à base de phosphate de calcium est toujours effectuée avec des composés purifiés, ladite purification étant effectuée à l'aide d'un protocole ne mettant pas en oeuvre de support à base de phosphate de calcium.

L'un des objectifs essentiels de l'invention est de simplifier le procédé de préparation d'antigènes tumoraux destiné à les mettre sous une forme reconnaissable par le système immunitaire afin de pouvoir l'appliquer à grande échelle par un personnel n'ayant pas une qualification de biochimiste.

Pour atteindre cet objectif, les inventeurs ont eu le mérite de développer un procédé de préparation en une seule étape à partir du broyat tumoral qui permet de disposer d'un vaccin sur un matériau vecteur. Ce matériau permet à la fois, la purification, la vectorisation dans l'organisme et le transport des substances actives dans les cellules cibles : les cellules présentatrices d'antigènes (APCs).

Pour atteindre cet objectif, les inventeurs ont eu le mérite de mettre en évidence l'intérêt de certains supports minéraux microparticulaires biocompatibles aptes à adsorber des molécules et/ou des matériaux biologiques d'intérêt dans le but de séparer les molécules et les vectoriser.

Un autre objectif essentiel de l'invention est de proposer un nouveau médicament d'immunothérapie (par exemple autologue) pour le traitement des cancers, des maladies infectieuses ou des maladies auto-immunes, entre autres.

C'est ainsi que l'invention concerne un procédé de préparation d'antigènes tumoraux destiné à les mettre sous une forme reconnaissable par le système immunitaire caractérisé en ce qu'il consiste essentiellement à :
- mettre en oeuvre un extrait tumoral,
- mettre en contact cet extrait tumoral avec un support minéral particulaire apte à adsorber sélectivement les antigènes tumoraux visés et à les vectoriser *in vivo* à titre de substances actives sur le système immunitaire,
- faire en sorte que le support minéral particulaire adsorbe sélectivement les antigènes tumoraux d'intérêt,
- séparer le support minéral de l'extrait tumoral non adsorbé de manière à recueillir les antigènes tumoraux d'intérêt sous forme purifié et adsorbé sur le support.

Le procédé selon l'invention comprend avantageusement une étape supplémentaire de mélange des antigènes tumoraux purifiés et adsorbés sur le support avec au moins un cofacteur apte à promouvoir l'action des antigènes tumoraux sur le système immunitaire, ledit cofacteur étant préférentiellement issu d'extraits tumoraux, notamment de broyats, lyophilisats, dialysats ou culot de centrifugation.

Le cofacteur, quant à lui, provient avantageusement, au moins en partie, de l'extrait tumoral et plus particulièrement encore de l'extrait à partir duquel les antigènes tumoraux visés ont été purifiés par adsorption sur le support minéral particulaire. Ce cofacteur est plus avantageusement encore choisi parmi les cytokines, interleukines, facteur de croissance ou interféron ou leurs mélanges.

Il est à noter que les moyens et le procédé selon l'invention permettent l'élimination des facteurs tumoraux pouvant entraîner des effets secondaires.

Dans une mise en oeuvre particulière de l'invention, le procédé de préparation des protéines tumorales comprend les étapes suivantes :
- préparation de l'extrait tumoral permettant la mise en suspension ou la solubilisation des antigènes tumoraux d'intérêt,
- percolation de la suspension ou de la solution à travers au moins une colonne contenant le support minéral particulaire,
- lavage de la colonne par des solutions tampon de force ionique et pH déterminés,
- collecte du support ayant adsorbé les antigènes tumoraux.

L'étape de lavage de la colonne permet de séparer le support minéral de l'extrait tumoral non nécessaire à l'immunogénicité. Elle est avantageusement effectuée à plusieurs reprises avec des tampons phosphate ou de solution saline de concentration croissante. Ainsi, le premier lavage est effectué avec un tampon phosphate ou une solution de NaCl de concentration inférieure ou égale à 200 mM alors qu'un deuxième lavage peut être effectué avec un tampon phosphate ou une solution de NaCl de concentration comprise entre 300 à 500 mM.

En pratique, l'étape de préparation de l'extrait tumoral est avantageusement effectuée de la façon suivante :
- éventuellement congélation du tissu tumoral,
- broyage du tissu tumoral,
- solubilisation ou mise en suspension des antigènes tumoraux cytoplasmiques dans une solution de NaHCO₃,
- centrifugation,
- séparation du culot et du surnageant.

Toujours en pratique, lorsque le procédé de préparation de antigènes tumoraux met en oeuvre l'étape supplémentaire de mélange des antigènes tumoraux purifiés et adsorbés sur le support minéral microparticulaires avec un cofacteur, ledit cofacteur est avantageusement préparé à partir du culot de centrifugation obtenu lors de la préparation de l'extrait tumoral. Ainsi, ledit culot est mis en suspension, il subit une étape visant à séparer de la fraction membranaire par centrifugation dans un gradient de sucre, de préference de saccharose, la fraction membranaire comprenant le cofacteur ainsi purifié étant récupérée.

Le procédé selon l'invention repose sur la mise en évidence par les inventeurs de l'intérêt de certains supports particuliers. Ainsi, le support minéral particulaire selon l'invention a des propriétés de surface autorisant, y compris sans agent couplant, la fixation spécifique des substances actives, notamment de l'agent biologique, et plus particulièrement d'antigènes tumoraux et leur transport vers des cellules du système des phagocytes mononucléés. Pour cela, le support minéral particulaire est choisi parmi les minéraux présentant tout ou partie des propriétés suivantes : présence d'au moins l'un des groupes ionisés ou ionisables POₐ²⁻, OH⁻ et/ou CA²⁺ à la surface dudit matériau, pH de surface basique, potentiel électrocinétique négatif et/ou hydrophobe. De tels composés peuvent être choisis dans le groupe comprenant les minéraux calciques, de préférence les phosphates de calcium et plus préférentiellement encore les hydroxyapatites. Le support minéral microparticulaire de choix répondant aux caractéristiques précitées est choisi parmi les céramiques et notamment une céramique de phosphate de calcium, en particulier une hydroxyapatite, un phosphate tri, tétra ou octocalcique sous forme particulaire.

Les céramiques sont un matériau de choix pour la mise en oeuvre du procédé selon l'invention, en effet, les phosphates de calcium ayant subi un traitement thermique calibré sont plus adaptés à la mise en contact, et particulièrement percolation d'un broyat de tissus que les phosphate de calcium directement issus de la synthèse sans traitement. Les broyats de tissus contiennent notamment des fibrines, lesquelles sont susceptibles de polymériser en présence de calcium et donc de colmater une colonne comprenant un tel support.

Un tel support sous certaines conditions de forme et de granulométrie permet la percolation dans une colonne d'un broyat tumoral sans colmatage de ladite colonne.

L'invention est par ailleurs également en partie fondée sur le constat que ces microparticules ayant adsorbé des substances actives ou aptes à le faire, sont "phagocytables", lorsque la taille desdites microparticules est suffisamment faible, par des cellules appartenant au groupe des phagocytes mononucléés qui comprend les macrophages, cellules dendritiques et autres cellules présentatrices de l'antigène (APCs). Une autre observation inventive faite par les inventeurs, est que l'introduction in vivo de ces particules de taille phagocytable dans le tissu sous cutané attire des cellules du système des phagocytes mononucléés et notamment les cellules présentatrices de l'antigène (APC), qui se concentrent là où se trouvent les microparticules du médicament selon l'invention et qui les phagocytent.

Ainsi, la taille des particules du support minéral particulaire est une caractéristique importante de l'invention. Suivant une disposition préférée, il est judicieux que la granulométrie du support minéral particulaire soit inférieure ou égale à 200 µm, de préférence inférieure ou égale à 90 µm, et, plus préférentiellement encore inférieure ou égale à 50 µm, de façon tout à fait préférée comprise entre 10 et 50 µm, idéalement inférieure à 10 µm et de surface spécifique comprise entre 0,1 et 5 m²/g, de préférence entre 0,3 et 2 m²/g. Il est important de préciser que, du fait de la dégradabilité du support et de sa structure, les particules vont se fractionner en microparticules de taille inférieure une fois injectées dans le malade.

Par "granulométrie", on entend taille moyenne des particules, et plus précisément moyenne de la plus grande dimension des particules non sphériques ou diamètre pour des particules sphériques. Les méthodes de mesure de la granulométrie sont conventionnelles, par exemple par diffraction laser.

Ces caractéristiques de granulométrie concernent plus spécialement mais non limitativement des supports solides pulvérulents, de préférence à base d'hydroxyapatite. La forme des particules est également importante, en effet, les particules de forme sphériques sont particulièrement adaptées à la mise en oeuvre selon l'invention, contrairement aux particules en forme d'aiguille.

Selon une variante, le procédé selon l'invention comprend une étape supplémentaire effectuée après la purification et la fixation sur le support selon l'invention, et selon laquelle les antigènes tumoraux sont désorbés dans une solution de NaCl ou un tampon phosphate 500 mM qui est ensuite diluée entre 100 et 200 mM repassée sur une colonne comprenant un support tel que défini aux revendications 10 à 13 de granulométrie inférieure à 10 µm.

Selon une variante, le support solide peut être non pulvérulent. Dans un tel cas, il peut s'agir par exemple d'une céramique apte à se dégrader in vivo par dissolution des joints de grains et relargage de particules constituées d'un nombre variable de grains. Lorsque leur taille est inférieure ou égale à environ 200, voire 90 et mieux encore 50 µm, les particules sont phagocytables par des macrophages ou des cellules géantes qui s'accumulent dans les zones de dégradation de la céramique.

Le support solide (par exemple céramique), peut, après avoir été dégradé sous forme de grains, pénétrer dans les cellules par pino-, endo- et phagocytose pouvant transporter des molécules biologiques et en particulier des antigènes tumoraux et des facteurs adjuvants.

Le support solide peut être par exemple composé de l'association d'une céramique et d'un ou plusieurs polymères biocompatibles et dégradables. Il peut également s'agir d'un solide comme décrit avec à sa surface des molécules organiques permettant de fixer des antigènes à la surface et/ou de cibler certains types cellulaires.

Les matériaux biologiques adsorbables ou adsorbés comme antigènes sont avantageusement choisis dans les constituants des cellules tumorales, les molécules, les organites, les produits de transformation des cellules (e.g. broyats, lyophilisats, dialysats, culot de centrifugation...) ainsi que les constituants des virus, bactéries, endototoxines et leurs mélanges.

De préférence, les antigènes, de préférence tumoraux - naturels ou synthétiques, modifiés ou non chimiquement et/ou physiquement et/ou génétiquement entre autres - sont sélectionnés parmi les antigènes membranaires, les antigènes cytoplasmiques, les antigènes extracellulaires et leurs mélanges.

Les molécules biologiques adsorbables ou adsorbées sont avantageusement choisies dans le groupe des facteurs adjuvants suivant : les protéines - de préférence les protéines du coup de chaleur (HSP, "Heat Shock Proteins") - , les acides nucléiques, les lipides, les phospholipides, les glucides, les glycoprotéines.

Ces HSPs sont notamment mais non exclusivement la gp96, l'hsp70, l'hsp90 et l'hsp 100.Les substances actives adsorbées ou adsorbables sur le support biocompatible peuvent également comprendre des gènes codant pour les matériaux et/ou les molécules biologiques tels que définis ci-dessus, de préférence pour des antigènes tumoraux et/ou des facteurs adjuvants - de préférence des cytokines et/ou lymphokines - permettant d'activer les cellules présentatrices d'anticorps (APCs).

De préférence, les substances actives peuvent comprendre des antigènes tumoraux et/ou des gènes codant pour des antigènes tumoraux, et, plus particulièrement des protéines du coup de chaleur (HSP). Les molécules biologiques sont ainsi par exemple des antigènes tumoraux associés ou non à des HSP.

De façon tout à fait préférentielle, les antigènes tumoraux sont choisis parmi :
- les antigènes dont l'expression est partagée dans différents types histologiques de cancers et plus particulièrement les antigènes MAGE (melanoma antigens), BAGE (bladder antigens), GAGE (gastric antigens), RAGE (renal antigens), α-foetoprotein, MUCI, HER-2/neu et leurs mélanges,
- les antigènes codés par des gènes mutés et plus particulièrement les gènes Oncogènes ras, Gènes suppresseur p53, β-caténine, Cdk4, la fusion des antigènes bcr/abl, produits de gènes viraux (Epstein Barr, hépatite B, papillome), CASP-8 et leurs mélanges,
- les antigènes associés aux tumeurs Antigène carcino-embryonnaire, Antigène prostatique, RU2, Alt-M-CSF, Tyrosinase, Melan-A/MART1, Gp100, Gp75, Glioma associated oncogen, GLIP1, et/ou
- des protéines du coup de chaleur (HSP, Heat Shock Protein), de préférence la protéine gp96 et les peptides associés.

L'invention concerne également un médicament, de préférence un autovaccin, caractérisé en ce qu'il comprend des antigènes tumoraux obtenus par le procédé décrit ci-dessus. Ainsi, le médicament selon l'invention comprend au moins un support solide biocompatible, de préférence en poudre, sur lequel est adsorbée ou sur lequel est destinée à être adsorbée, de préférence sans nécessiter d'agent couplant, au moins une substance active, de préférence choisie parmi les matériaux biologiques comprenant le groupe des antigènes contenus dans les cellules tumorales, leurs organites cellulaires, leur membranes, leurs produits de transformation (broyats, lyophilisats, dialysats, culot de centrifugation...), les virus, les bactéries, anticorps, endotoxines et leur mélange, ledit support biocompatible ayant servi à purifier ladite substance active.

Par médicament, il faut comprendre que les dispositifs médicaux, comprenant par exemple une poudre minérale et au moins une substance active, sont également couverts. On nomme agent couplant une fraction chimique qui relie de façon covalente le support solide biocompatible et la substance active. Il peut notamment s'agir d'une liaison covalente ou d'un radical organique.

L'invention est en partie fondée sur le constat que ces microparticules ayant adsorbé des substances actives ou aptes à le faire sont « phagocytables », lorsque la taille desdites microparticules est suffisamment faible, par des cellules appartenant au groupe des phagocytes mononucléés qui comprend les macrophages, cellules dendritiques et autres cellules présentatrices d'antigènes (APCs). Une autre observation inventive faite par les inventeurs est que l'introduction in vivo de ces particules de taille phagocytable dans le tissu sous cutané attire des cellules du système des phagocytes mononucléés et notamment les APCs, qui se concentrent là où se trouvent les microparticules du médicament selon l'invention et qui les phagocytent.

Les inventeurs ont mis en évidence le fait qu'une molécule adsorbée sur une particule, par exemple d'HA, de taille phagocytable, pénètre rapidement e.g. dans les APCs. Sans que cela ne soit limitatif, cette pénétration rapide peut s'expliquer de deux manières : soit la molécule adsorbée est directement libérée dans la cellule après avoir été phagocyté avec la particule support, soit cette molécule est désorbée et libérée dans les environs immédiats de la cellule et y pénètre dans un second temps.

Par exemple, lorsque la protéine du coup de chaleur HSP gp 96 est fixée sur les particules d'HA, elle peut en être libérée à l'aide d'une solution de phosphate de calcium titrant 200-300 mM, ce qui est une concentration rencontrée in vivo dans les liquides intra et extracellulaires.

Avantageusement, ce médicament peut être utilisé pour des traitements autologues par immunothérapie, en particulier comme vaccin, de préférence comme vaccin anti tumoral. Selon une modalité remarquable de l'invention, les antigènes tumoraux sont préparés à partir de la tumeur du patient, le médicament étant alors un autovaccin.

Selon l'une des modalités particulièrement surprenante de l'invention, le médicament comprend des antigènes tumoraux adsorbés sur un support minéral particulaire ayant servi à la purification desdits antigènes à partir d'un extrait tumoral.

Les substances actives, matériaux biologiques et plus particulièrement les agents tumoraux adsorbables ou adsorbés sur le support biocompatible et qui constituent pour partie le médicament selon l'invention sont ceux qui ont été définis ci-avant.

Dans une autre mise en oeuvre de l'invention, le médicament peut comprendre outre les protéines tumorales adsorbées sur le support minéral microparticulaire, un cofacteur ledit cofacteur étant préférentiellement sous la forme d'extraits tumoraux, notamment de broyats, lyophilisats, dialysats ou culot de centrifugation. De façon tout à fait préférentielle, le cofacteur provient au moins en partie d'un extrait tumoral, et plus particulièrement de l'extrait tumoral à partir duquel les protéines tumorales visées ont été purifiées par adsorption sur le support minéral particulaire.

Le médicament selon l'invention comporte des particules solides biocompatibles, de préférence, de phosphates de calcium, qui ont de manière surprenante et inattendue la propriété de stimuler la croissance et les performances des macrophages et/ou d'autres cellules APC, telles que les cellules dendritiques.

L'invention vise donc également un médicament caractérisé en ce qu'il est destiné à activer des macrophages et/ou d'autres cellules APC, c'est-à-dire entraîner la synthèse de substances choisies dans le groupe comprenant les cytokines, les lymphokines, les facteurs de croissance, et/ou rendre les cellules dendritiques matures.

Une autre caractéristique avantageuse du médicament tient à ce qu'il est destiné à être phagocyté par des macrophages et/ou d'autres cellules APC et/ou des cellules dendritiques.

Le médicament selon l'invention est également remarquable en ce qu'il permet le transport in vivo d'une ou plusieurs substances actives.

En d'autres termes, le support solide (par exemple phosphates de calcium, en poudre ou non -massif-, céramiques ou non) selon invention permet de vectoriser toute substance/molécule adsorbable, en utilisant les cellules présentatrices de l'antigène APCs ou autres cellules analogues.

S'agissant de sa mise en oeuvre, le médicament selon l'invention est caractérisé en ce qu'il est implantable in vivo et/ou administrable par injection.

Suivant une modalité intéressante, le médicament selon l'invention permet la libération prolongée (par exemple pendant quelques heures à plusieurs jours) in vivo de la (ou des) substances active(s) adsorbée(s).

Une fois injecté dans un tissu conjonctif, le médicament selon l'invention peut entraîner un afflux local de macrophages, de cellules dendritiques et/ou d'autres cellules APC présentatrices de l'antigène ou des antigènes adsorbé(s) sur le support.

L'adsorption de la (ou des) substance(s) active(s) sur le support solide selon l'invention peut s'opérer in vivo et/ou ex vivo, ce support étant une poudre, de préférence de l'hydroxyapatite telle que définie ci-dessus.

Ainsi, un médicament comprenant un support solide en poudre selon l'invention permet la percolation à travers une colonne dudit support d'un broyat tumoral et l'injection de ladite poudre dans le tissu sous cutané ou bien les ganglions lymphatiques.

En pratique, le médicament selon l'invention comprend des antigènes tumoraux dont la quantité est directement proportionnelle à la surface spécifique de la poudre. De façon préférentielle le médicament comprend des antigènes tumoraux à hauteur de 20 mg/g de poudre de support minéral.

Lorsqu'il est employé sous la forme d'une unité galénique, le médicament selon l'invention est avantageusement employé en quantité comprise entre 15 et 100 µg dose/unité galénique.

Le support solide mis en oeuvre dans le médicament suivant l'invention, peut servir à la fois pour purifier des substances actives, immobiliser à sa surface des substances actives, injecter/implanter et libérer des substances actives dans l'organisme ou convoyer des substances actives biologiques dans les APCs ou autres cellules analogues.

L'invention a également pour objet l'utilisation du support solide biocompatible, de préférence en poudre, tel que défini ci-dessus, et, éventuellement d'au moins une substance active également définie supra, pour préparer un médicament tel que défini ci-dessus.

Un autre objet de l'invention vise un procédé de préparation d'un médicament, en particulier tel que décrit ci-avant, caractérisé en ce qu'il consiste essentiellement à mettre en contact au moins un support solide biocompatible, de préférence en poudre, avec au moins une substance active, de préférence choisie parmi les matériaux biologiques et/ou les molécules biologiques, de manière à ce que la substance active s'adsorbe réversiblement sur le support.

De préférence, ce procédé est caractérisé en ce qu'il comprend la purification de substances actives, éventuellement autologues, pouvant être utilisées pour provoquer une immunothérapie, ce procédé comprenant les étapes essentielles suivantes :
• Mettre en contact de la ou des substances actives avec une poudre de phosphate de calcium pendant un temps suffisant pour que se produise l'immobilisation/adsorption d'au moins une partie de la ou des substances actives
   - de préférence au moins un facteur immunitaire et/ou au moins un facteur adjuvant - sur la poudre ;
■ Récupérer la poudre ayant immobilisé au moins une partie de la ou des substances actives, pour préparer un médicament administrable à un patient atteint par exemple d'un cancer, d'une maladie infectieuse ou d'une maladie auto-immune, cette ou ces substances actives pouvant provenir du patient dans le cas d'un traitement autologue.

Dans une variante, ce procédé est caractérisé en ce qu'il comprend la purification de facteurs autologues pouvant être utilisés pour provoquer une réaction immunitaire antitumorale, ce procédé comprenant les étapes essentielles suivantes :
■ Mise en contact d'un broyat de tumeur avec une poudre de phosphate de calcium pendant un temps suffisant pour que se produise l'immobilisation de certains facteurs moléculaires - de préférence au moins un facteur immunitaire et/ou au moins un facteur adjuvant - sur la poudre ;
■ Laver la poudre avec une ou plusieurs solutions salines de diverses molarités et/ou pH pour éliminer les molécules non adsorbées.
■ Récupérer la poudre ayant immobilisé certains facteurs moléculaires pour préparer un médicament injectable dans l'organisme de l'individu atteint de la tumeur dont est extrait le broyat.

En pratique, la mise en contact peut être, par exemple, une purification réalisée à l'aide d'une ou plusieurs colonne séparées ou pas par un système de réservoir dans lequel peuvent être introduites ou soustraites certaines solutions et au travers de laquelle ou desquelles on peut faire percoler un broyat de tumeur.

Toujours en pratique, la récupération de la (ou des) substances active(s) peut, par exemple, s'opérer par élution de la (ou des) colonne(s) au moyen d'une solution de tampon - de préférence de tampon phosphate- de molarité et de pH appropriés, l'éluat ainsi obtenu comprenant des antigènes tumoraux et/ou les facteurs adjuvants immobilisés et recherchés. Il s'agit ici d'une technique de chromatographie.

Dès lors qu'il ne s'agit pas de chromatographie, la récupération de la (ou des) substance(s) active(s) s'opère par récupération de la poudre ayant adsorbé les antigènes tumoraux et/ou les facteurs adjuvants immobilisés et recherchés.

Grâce aux propriétés d'adsorption sélective du support solide utilisé suivant l'invention, il est possible d'effectuer un dosage de substances actives adsorbées après désorption.

Ce dosage peut par exemple être réalisé par contrôle de la surface spécifique du support mesurée par adsorption gazeuse (mesure BET). Cependant, toutes les méthodes d'analyse connues sont envisageables.

Pour illustrer cette faculté d'adsorption sélective, on peut signaler que la gp96, par exemple, se fixe à la surface des particules d'HA à des molarités de tampon phosphate faibles. Elles sont désorbées des poudres entre 200 mM et 300 mM.

L'invention vise en outre et entre autres :
■ un procédé de préparation d'un médicament caractérisé en ce qu'il comprend le procédé de préparation d'antigènes tumoraux tel que décrit ci-avant. De façon préférentielle, ce procédé met en oeuvre un extrait tumoral préparé à partir de la tumeur du patient, ledit médicament étant un auto vaccin. Selon une mise en oeuvre particulière dudit procédé les antigènes tumoraux purifiés adsorbés sur le support particulaire, éventuellement mélangés à au moins un cofacteur, sont sous la forme d'une préparation injectable.
■ un procédé de purification de molécules/matériaux biologiques du type de ceux ci-dessus défini, et en particulier les protéines de coup de chaleur HSPs et/ou les antigènes anti-tumoraux ; consistant à utiliser le support solide tel que défini ci-dessus comme moyen d'adsorption /désorption sélective ;
■ un système de purification contenant une poudre ci-dessus définie, et constitué d'une ou plusieurs colonnes séparées ou pas par un système de réservoir dans lequel peuvent être introduites ou soustraites certaines solutions et permettant à partir de l'introduction d'un broyat de tumeur dans le système l'obtention en sortie d'une poudre injectable sur laquelle sont adsorbés des antigènes tumoraux et/ou des facteurs adjuvants.

Ce système de purification comprend avantageusement un système d'introduction de la poudre dans l'organisme, amovible et permettant de l'injecter ou la projeter dans le tissu sous-cutané du patient.
■ des méthodes thérapeutiques consistant à administrer le médicament selon l'invention pour le traitement et/ou la prévention notamment de cancers, de maladies infectieuses ou de maladies auto-immunes ;
■ des méthodes thérapeutiques consistant à administrer le médicament selon invention pour le traitement et/ou la prévention par immunothérapie (par exemple autologues), notamment de cancers, de maladies infectieuses ou de maladies auto-immunes ;
■ et des vaccins autologues comprenant le médicament selon l'invention pour le traitement et/ou la prévention notamment de cancers, de maladies infectieuses ou de maladies auto-immunes.

Il ressort notamment de l'exposé ci-dessus de l'invention que les particules d'HA sont un bon vecteur pour transporter les protéines du coup de chaleur à l'intérieur des macrophages et des cellules qui présentent les antigènes comme le montre les exemples suivants.

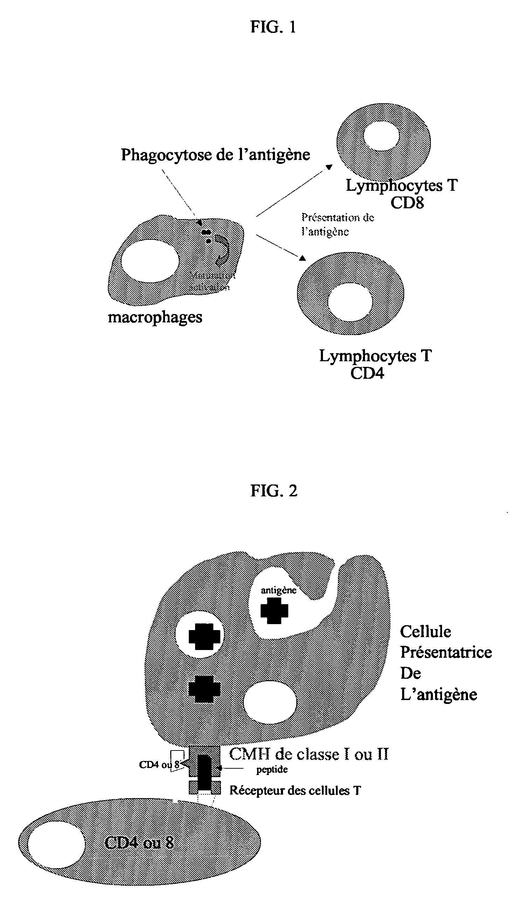

Fig. 1 : présentation de l'antigène par les APCs aux cellules effectrices de la réaction immunitaire, les lymphocytes T.
Fig. 2 : l'antigène une fois phagocyté est fragmenté en peptides qui sont associés au CMH pour pouvoir être reconnus par les lymphocytes.
Fig. 3 A : coupe histologique de particules de phosphates de calcium implantées dans une mandibule de lapin portant un plasmide vecteur d'un gène Lac-Z montrant une accumulation de macrophages et d'APCs autour des particules.
Fig. 3B : coupe histologique de particules de phosphates de calcium implantées dans une mandibule de lapin portant un plasmide vecteur d'un gène Lac-Z montrant une accumulation de macrophages et d'APCs autour des particules. La révélation de l'activité galactosidase montre que toutes les cellules de proximité sont transfectées.
Fig. 4 : coupe en microscopie électronique à transmission de cellules médullaires cultivées au contact de particules d'HA après déminéralisation. Il existe de très nombreuses vésicules cytoplasmiques vides qui contenaient les particules avant leur dissolution.
Fig. 5 : mRNA de TNF-α/mRNA d'actine β
Fig.6 : mRNA de IL-6/mRNA de β actine
Fig. 7 : slot blot de différentes fractions de broyat de tumeurs recueillies à la sortie d'une colonne d'HA. La fraction obtenue par lavage à 200 mM de tampon phosphate montre le meilleur rendement en gp96 (HSP) (barre noire en haut de l'électrophorèse).
Fig. 8 : western blot de la fraction 200 mM

### Exemple 1 :

### Capacité de transport intracellulaire par des particules d'HA de molécules biologiques à l'intérieur des macrophages et cellules géantes :

2 mg de particules d'HA sont utilisés dans cette expérimentation. Les caractéristiques de la poudre sont les suivantes : granulométrie 45-80µm ; surface spécifique : 0,7 m²/gr ; forme sphérique ; charge de surface négative, potentiel de surface -37 mV, hydrophobes. Un plasmide portant un gène de la galactosidase (Lac-Z) est adsorbée à la surface des poudres suivant le protocole suivant : 25 µg de plasmide sont dilués dans 2 ml de tampon phosphate pH 6,8, 0,1 M. La poudre est incubée deux heures dans la solution de plasmide à 37°C puis séchée.

Les 2 mg de poudre son implantés dans un défect osseux effectué à la jonction du ligament des incisives de mandibules de lapins. Les animaux son sacrifiés à trois semaines et les mandibules prélevées. Des coupes histologiques sont effectuées et l'activité galactosidase des cellules mises en évidence par la réaction à la GalX avant d'être observées en microscopie optique.

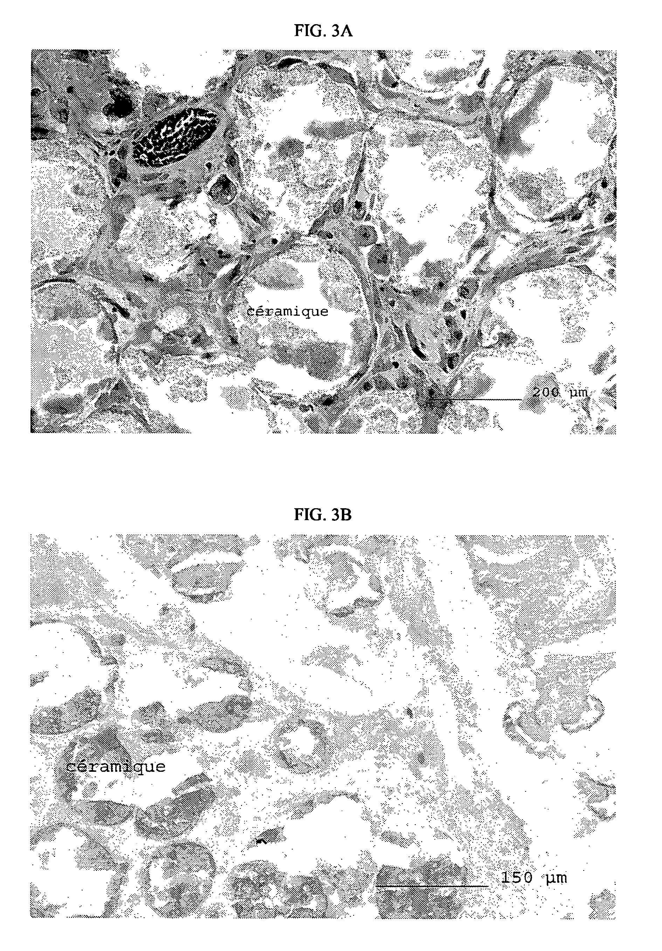

Il existe une accumulation de macrophages et cellules géantes autour des particules de poudre (fig. 3A et 3B). Toutes les cellules en périphérie des poudres ont une activité galactosidase ce qui suggère que le plasmide a migré de la surface des poudres à l'intérieur du noyau des macrophages. Des fragments de particules sont visibles dans les cytoplasmes ce qui indique qu'ils ont été phagocytés. Il y a de nombreuses cellules mononucléées ou plurinucléées exprimant le gène lac-Z qui ont migré à la surface des trabécules à distance des particules.

Cette expérience montre que :
• l'ADN immobilisé à la surface des particules de HA est transporté préférentiellement à l'intérieur des cellules qui présentent l'antigène.
• ces cellules migrent avec les molécules qu'elles contiennent

### Exemple 2 :

### Dégradation des particules de poudre :

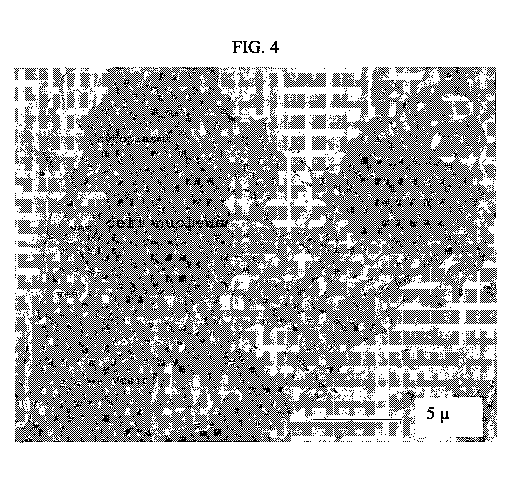

10 mg de poudres stériles avec les caractéristiques de l'exemple 1 sont introduites dans un flacon de culture qui contient une couche confluente d'une lignée primaire de cellules médullaires humaines. Les cellules sont cultivées au contact des poudres dans un milieu DMEM supplémenté en 10% de sérum de veau foetal et glutamine à 37°C dans une atmosphère à 5% de CO₂ durant 4 jours. Elles sont ensuite examinées en microscopie optique. Elles manifestent une activité phosphatase acide indiquant que ces cellules appartiennent au système des phagocytes mononucléés. De nombreuses particules ou fragments de céramiques sont au contact ou inclus dans le cytoplasme. Les cellules sont ensuite fixées dans une solution de 4% de glutaraldéhyde, déminéralisées dans une solution d'EDTA, incluses dans un polymère epoxy. Des coupes de 200 angströms sont faites et examinées en microscopie électronique à transmission. Il y a de très nombreuses vésicules vides dans le cytoplasme cellulaire qui indiquent l'emplacement des particules avant déminéralisation (fig. 4).

Il est donc avéré que les particules d'HA se dégradent en émettant des grains qui sont phagocytés par les cellules présentant les antigènes.

### Exemple 3 :

### Activation des APCs par les particules :

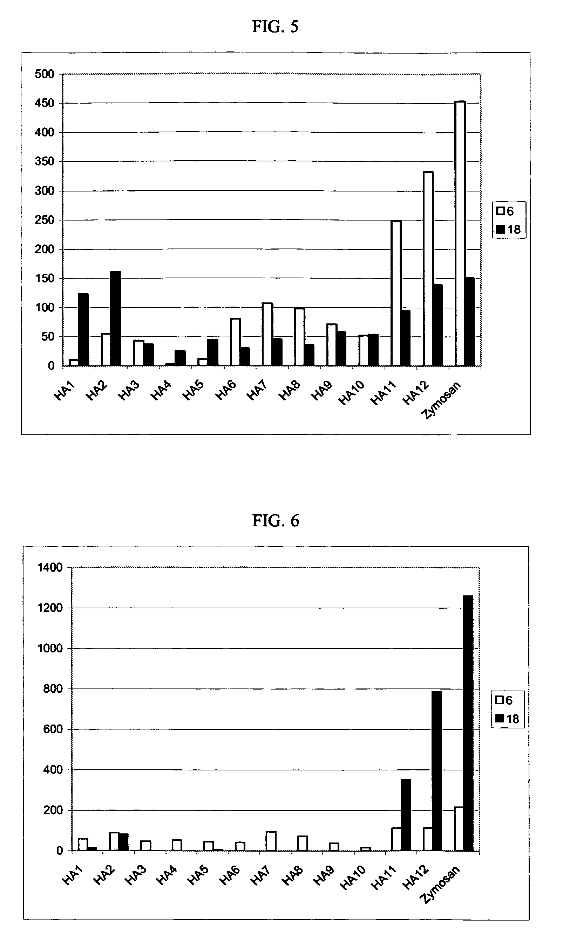

Diverses particules d'hydroxyapatite sont introduites dans un flacon de culture contenant une lignée primaire de monocytes humains avant la confluence. Les cellules sont en contact avec les poudres, dont les caractéristiques sont données dans le tableau 2, pour 6 ou 18 heures. La densité cellulaire à l'ensemencement des boîtes de culture est de 2.10⁵ cellules/ml. Le milieu de culture est du RPMI-1640 supplémenté en 10% de sérum de veau foetal. Les cellules sont cultivées à 37°C et dans une atmosphère à 5% de CO₂. La masse de poudre introduite dans la culture est la masse nécessaire pour que le rapport superficie des cellules/superficie de la poudre soit égal à 1. Les quantités d'ARNm de TGF-β et d'IL-6 sont dosées à 6 et 18 heures (fig. 5 et 6).

**Tableau 2 : caractéristiques des poudres :**

| | HA1 | HA2 | HA3 | HA4 | HA5 | HA6 | HA7 | HA8 | HA9 | HA10 | HA11: | HA12 |
|---|---|---|---|---|---|---|---|---|---|---|---|---|
| Granulométrie (µm) | 1-30 | 1-30 | 10-70 | 110-190 | 170-300 | 1-30 | 1-30 | 1-30 | 100-250 | 150-300 | 1-30 | 1-30 |
| Taille moyenne (µm) | 3 | 3 | 44 | 140 | 217 | 3 | 3 | 3 | 166 | 196 | 2 | 2 |
| Frittage (°C) | 600 | 1180 | 1180 | 1180 | 1180 | 20 | 600 | 1180 | 1180 | 1180 | 20 | 600 |
| Surface spécifique (m²g⁻¹) | 23.95 | 5.38 | 1.56 | 0.63 | 0.5 | 38.06 | 26.72 | 6.08 | 0.7 | 0.7 | 18.51 | 13.18 |
| Taille des grains (nm) | 190 | 350 | 350 | 350 | 350 | 80 | 180 | 350 | 420 | 350 | 200 | 300 |
| Forme (1) | S | S | S | S | S | Q | Q | Q | Q | Q | A | A |

| | | | | | | | | | | | | |
|---|---|---|---|---|---|---|---|---|---|---|---|---|
| (1) Forme des grains : S = Sphère ; Q = Quelconque ; A = Aiguille | | | | | | | | | | | | |

Les résultats montrent que les caractéristiques des particules d'hydroxyapatite influent leur aptitude à faire synthétiser des interleukines et du TGF-β par les APCs qui les ont phagocytées c'est à dire à les activer. Les particules en forme d'aiguille sont les plus inflammatoires. En ce qui concerne les autres formes, les particules les plus petites sont les plus inflammatoires. Il est connu que les particules de poudre d'HA se dégradent durant leur implantation tissulaire et que des débris plus petits sont émis, ce qui permet d'amplifier l'activation des macrophages.

### Exemple 4 :

### Purification de protéines du coup de chaleur gp96 sur des lits de poudre d'HA injectable

La purification est effectuée selon les étapes suivantes :

### Préparation du support :

10 gr de poudre sont réhydratés huit heures dans un tampon phosphate de basse force ionique (0,01 M, pH 6,8) à température ambiante dans un volume égal à 4 à 5 fois le volume de la poudre. L'excès de tampon est enlevé avec les particules qui n'ont pas sédimenté. La poudre est remise en suspension dans un excès de tampon, versée dans la colonne dont l'extrémité inférieure est fermée. Elle est laissée décanter pendant quelques heures et le tampon est ensuite évacué par le bas de la colonne. La colonne est ensuite équilibrée dans un tampon phosphate (0,01M, pH 6,8). Puis la colonne est lavée dans un tampon phosphate 30 mM, pH 6,8 (20-30 fois le volume de la colonne).

### Percolation de la suspension à travers la colonne contenant l'HA :

La charge de protéine (précipité du broyat de tumeur additionné de tampon phosphate 30 mM, pH 6,8 dans une proportion de 1/10) est introduite dans la colonne.

### Lavage de la colonne :

La colonne est ensuite lavée par un gradient de tampon phosphate de 0 à 500 mM.

### Collecte du support et des fractions protéiques.

### Exemple 5 :

### Contrôle des fractions protéiques obtenues par fractionnement sur colonne d'HA à différentes molarités de tampons phosphate :

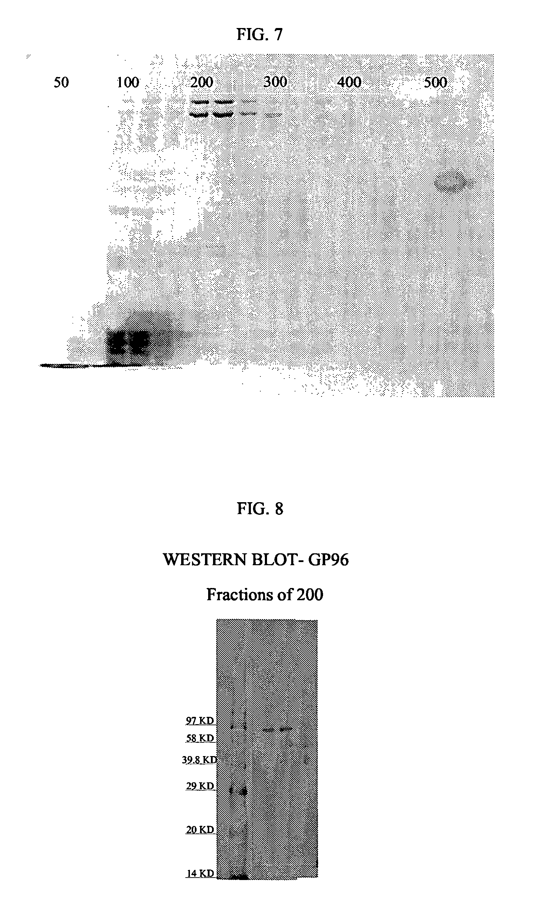

Deux contrôles des diverses fractions ont été effectués, par des méthodes d'électrophorèse slot blot et western blot qui sont des méthodes bien connues. Les résultats de l'électrophorèse montrent que la majorité des protéines gp96 se détachent de l'HA à 200 mM. Le western blot montre que la fraction protéique est quasiment pure à cette molarité (figures 7 et 8).

### Exemple 6 :

### Préparation d'un autovaccin

Toutes les manipulations suivantes sont effectuées en conditions stériles.

### 1. Préparation du tissu tumoral et congélation.

Le tissu tumoral est prélevé (environ 1 cm³) lors d'une biopsie réalisée en conditions stériles et immédiatement congelé.

### 2. Broyage de l'extrait tumoral

Le tissu tumoral est broyé dans un mortier puis les fragments sont passés dans un tube de Kahn sur un lit de glace.

### 3. Dilution du broyat dans une solution de NaHCO₃

Le broyat obtenu l'étape 2 est mélangé avec 750 µl de NaHCO₃, 30mM, pH7. Le tissu est ensuite homogénéisé avec un broyeur à couteaux.

### 4. Séparation du culot et du surnageant par centrifugation

L'homogénat est transféré dans un tube Eppendorf et centrifugé entre mille et deux mille g durant 30 mn à 4°C. Deux fractions sont alors obtenues, l'une d'elle correspond au surnageant comprenant les protéines tumorales en suspension et en solution, l'autre correspond au culot et comprend les fractions membranaires.

### 5. percolation de la suspension sur une colonne d'hydroxyapatite (HA).

### Préparation de la colonne :

Une colonne de poudre d'HA est préparée : la poudre est mise en suspension dans un tampon phosphate (20 mM, pH7), laissée sédimenter 30 secondes et le surnageant jeté afin d'éliminer les particules fines en suspension susceptibles de colmater la colonne lors du passage de la solution de protéines. La colonne est ensuite lavée avec 10 fois le volume représenté par la poudre par une solution de tampon phosphate.

### Percolation du surnageant sur le colonne d'HA:

Les protéines du surnageant obtenu à l'étape 4 sont précipitées par une solution de sulfate d'ammonium et le précipité est repris dans une solution de tampon phosphate dans lequel les protéines sont solubilisées ou en suspension. La solution contenant les protéines tumorales en suspension et solubilisées est percolée sur lit de céramiques afin de récupérer les protéines du coup de chaleur (gp96).

### 1. Lavage de la colonne

Lorsque la solution de protéines a pénétré dans le lit d'HA, la colonne est lavée avec 4 à 5 fois son volume de tampon phosphate (20mM, pH7). La colonne est ensuite lavée par un gradient de tampon phosphate ou de chlorure de calcium de 0-200 mM de 4 à 5 fois le volume de la poudre.

### 2. Préparation du cofacteur

Le culot obtenu à l'étape 4 est remis en suspension dans 400 µl de tampon phosphate (20 mM, pH 7).

Un gradient de saccharose est préparé par tranches de 400 µl, 40, 35 et 30% sur lequel sera déposé 200 µl du culot obtenu à l'étape 1. Le gradient ainsi chargé en extrait tumoral sera ensuite centrifuger à 1500 g pendant 30 mn à 4°C. La fraction contenant les fragments membranaires située à l'interface de saccharose 40/35% est récupérée.

### 3. Préparation du mélange à injecter.

La poudre en suspension ayant adsorbé les protéines du surnageant obtenue à l'issue de l'étape 4 est ensuite associée à la solution de membranes obtenue dans l'étape 3 (20 mg pour 100 µl). Le mélange est ensuite introduit dans une seringue où le volume du mélange est éventuellement adapté par l'adjonction d'une solution de NaCl (9/1000).

## Revendications

1. Procédé de préparation d'antigènes tumoraux, de préférence de protéines tumorales et de façon plus préférentielle encore de protéines tumorales autologues, destiné à les mettre sous une forme reconnaissable par le système immunitaire **caractérisé en ce qu'**il consiste essentiellement à :
a. mettre en oeuvre un extrait tumoral,
b. mettre en contact cet extrait tumoral avec un support minéral particulaire biocompatible solide, de préférence en poudre, apte à adsorber sélectivement les antigènes tumoraux visés et à les vectoriser *in vivo* à titre de substances actives sur le système immunitaire,
c. faire en sorte que le support minéral particulaire adsorbe sélectivement les antigènes tumoraux d'intérêt,
d. séparer le support minéral de l'extrait tumoral non adsorbé,
e. recueillir les antigènes tumoraux d'intérêt sous forme purifiée et adsorbée sur le support minéral particulaire.

2. Procédé selon la revendication 1 **caractérisé en ce que** l'extrait tumoral mis en oeuvre dans l'étape a. est un extrait tumoral autologue.

3. Procédé selon la revendication 1 ou 2 **caractérisé en ce qu'**il comprend une étape supplémentaire de mélange des antigènes tumoraux purifiés et adsorbés sur le support avec au moins un cofacteur apte à promouvoir l'action des antigènes tumoraux sur le système immunitaire, ledit cofacteur étant préférentiellement issu d'extraits tumoraux, notamment de broyats, lyophilisats, dialysats ou culot de centrifugation..

4. Procédé selon l'une des revendications précédentes **caractérisé en ce qu'**il comprend les étapes suivantes :
a. préparation de l'extrait tumoral permettant la mise en suspension ou la solubilisation des antigènes tumoraux d'intérêt,
b. percolation de la suspension ou de la solution à travers au moins une colonne contenant le support minéral particulaire,
c. lavage de la colonne par des solutions tampon de force ionique et pH déterminés,
d. collecte du support ayant adsorbé les antigènes tumoraux.

5. Procédé selon la revendication 4 **caractérisé en ce qu'**un premier lavage de la colonne est effectué avec un tampon phosphate ou une solution de NaCl de concentration inférieure ou égale à 200 mM et/ou qu'un deuxième lavage est effectué avec un tampon phosphate ou une solution de NaCl de concentration comprise entre 300 à 500 mM.

6. Procédé selon l'une des revendications 4 ou 5 **caractérisé en ce que** la préparation de l'extrait tumoral comprend les étapes suivantes :
a. éventuellement congélation du tissu tumoral,
b. broyage du tissu tumoral,
c. solubilisation ou mise en suspension des antigènes tumoraux dans une solution de NaHCO₃,
d. centrifugation,
e. séparation du culot et du surnageant.

7. Procédé selon l'une des revendications précédentes **caractérisé en ce que** le support minéral particulaire est choisi parmi les matériaux présentant tout ou partie des propriétés suivantes :
- présence d'au moins l'un des groupes ionisés ou ionisables PO₄²⁻, OH⁻ et/ou Ca²⁺ à la surface dudit matériau,
- pH de surface basique,
- charge de surface négative,
- hydrophobe.

8. Procédé selon l'une des revendications précédentes **caractérisé en ce que** le support minéral particulaire est choisi :
- dans le groupe comprenant les minéraux calciques, de préférence les phosphates de calcium et plus préférentiellement encore les hydroxyapatites, ou
- parmi les céramiques, de préférence une céramique de phosphate de calcium, en particulier une hydroxyapatite, un phosphate tri, tétra ou octocalcique sous forme particulaire.

9. Procédé selon l'une des revendications précédentes caractérisé en que les antigènes tumoraux comprennent :
a. des antigènes dont l'expression est partagée dans différents types histologiques de cancers et plus particulièrement les antigènes MAGE (melanoma antigens), BAGE (bladder antigens), GAGE (gastric antigens), RAGE (renal antigens), α-foetoprotein, MUCI, HER-2/neu et leurs mélanges,
b. des antigènes codés par des gènes mutés et plus particulièrement les gènes Oncogènes ras, Gènes suppresseur p53, β-caténine, Cdk4, la fusion des antigènes bcr/abl, produits de gènes viraux (Epstein Barr, hépatite B, papillome), CASP-8 et leurs mélanges,
c. des antigènes associés aux tumeurs Antigène carcino-embryonnaire, Antigène prostatique, RU2, Alt-M-CSF, Tyrosinase, Melan-A/MART1, Gp100, Gp75, Glioma associated oncogen, GLIP1, et/ou
d. des protéines du coup de chaleur (HSP, Heat Shock Protein), de préférence la protéine gp96 et les peptides associés.

10. Procédé selon l'une quelconque des revendications précédentes **caractérisé en ce qu'**après purification et fixation sur le support tel que défini aux revendications 7 ou 8, les antigènes tumoraux sont désorbés dans une solution de NaCl ou un tampon phosphate 500 mM qui est ensuite diluée entre 100 et 200 mM repassée sur une colonne comprenant un support tel que défini aux revendications 7 ou 8 de granulométrie inférieure à 10 µm.

## Claims

1. A method for preparing tumor antigens, preferably tumor proteins, and even more preferably autologous tumor proteins, intended to provide them in a form that can be recognized by the immune system, **characterized in that** it consists essentially in:
a. using a tumor extract,
b. bringing this tumor extract into contact with a biocompatible particulate mineral support capable of selectively adsorbing the targeted tumor antigens and in vectorizing them in *vivo* as substances that are active on the immune system,
c. seeing to it that the particulate mineral support selectively adsorbs the tumor antigens of interest,
d. separating the particulate mineral support from the nonadsorbed tumor extract,
e. collecting the tumor antigens of interest in the form that is purified and adsorbed onto the particulate mineral support.

2. The method according to claim 1, **characterized in that** the tumor extract that is used in step a is an autologous tumor extract.

3. The method according to claim 1 or 2, **characterized in that** it comprises an additional step of mixing the tumor antigens that have been purified and adsorbed onto the support, with at least one cofactor capable of promoting the action of the tumor antigens on the immune system, said cofactor being preferably derived from tumor extracts, in particular from ground materials, lyophilizates, dialysates or a centrifugation pellet.

4. The method as claimed in any one of the preceding claims, **characterized in that** it comprises the following steps:
a. preparing the tumor extract allowing the suspension or the solubilization of the tumor antigens of interest,
b. percolating said suspension or solution through at least one column containing the particulate mineral support,
c. washing the column with buffer solutions of given ionic strength and given pH,
d. collecting the support that has adsorbed the tumor antigens.

5. The method as claimed in claim 4, **characterized in that** a first wash of the column is carried out with a phosphate buffer or a solution of NaCl having a concentration of less than or equal to 200 mM and/or a second wash is carried out with a phosphate buffer or a solution of NaCl having a concentration of between 300 and 500 mM.

6. The method as claimed in 4 or 5, **characterized in that** the preparation of the tumor extract comprises the following steps:
a. optionally, freezing of the tumor tissue,
b. grinding of the tumor tissue,
c. solubilization or suspension of the tumor antigens in a solution of NaHCO₃,
d. centrifugation,
e. separation of the pellet and of the supernatant.

7. The method as claimed in one of the preceding claims, **characterized in that** the particulate mineral support is chosen from materials having all or some of the following properties:
- presence of at least one of the ionized or ionizable groups PO₄²⁻, OH⁻ and/or Ca²⁺ at the surface of said material,
- basic surface pH,
- negative surface charge,
- hydrophobic.

8. The method as claimed in one of the preceding claims, **characterized in that** the particulate mineral support is chosen:
- in the group comprising calcium minerals, preferably calcium phosphates, and even more preferably hydroxyapatites, or
- among ceramics, preferably a calcium phosphate ceramic, in particular a hydroxyapatite, or a tricalcium, tetra or octocalcium phosphate in particulate form.

9. The method as claimed in one of the preceding claims, **characterized in that** the tumor antigens comprise:
a. antigens whose expression is shared in various histological types of cancers, and more particularly the antigens MAGE (melanoma antigens), BAGE (bladder antigens), GAGE (gastric antigens), RAGE (renal antigens), α-fetoprotein, MUC1, HER-2/neu, and mixtures thereof,
b. antigens encoded by mutated genes, and more particularly the ras oncogenes, the suppressor genes p53, β-catenin and Cdk4, the bcr/ab1 antigen fusion, products of viral genes (Epstein Barr, hepatitis B, papilloma), CASP-8, and mixtures thereof,
c. the tumor-associated antigens carcinoembryonic antigen, prostate antigen, RU2, Alt-M-CSF, tyrosinase, melan-A/MART1, Gp100, Gp75, Glioma associated oncogene, GLIP1, and/or
d. heat shock proteins (HSPs), preferably the gp96 protein and the associated peptides.

10. The method as claimed in any one of the preceding claims, **characterized in that**, after purification and binding to the support as defined in claims 7 or 8, the tumor antigens are desorbed in a solution of NaCl or a phosphate buffer at 500 mM, which is then diluted to between 100 and 200 mM and again passed over a column comprising a support as defined in claims 7 or 8, having a particle size of less than 10 µm.

## Patentansprüche

1. Verfahren zur Herstellung von Tumorantigenen, vorzugsweise Tumorproteinen und noch bevorzugter autologen Tumorproteinen, das dafür bestimmt ist, sie in einer Form bereitzustellen, in der sie vom Immunsystem erkannt werden können, **dadurch gekennzeichnet, dass** es im Wesentlichen in Folgendem besteht:
a. Verwenden eines Tumorextrakts,
b. Kontaktieren dieses Tumorextrakts mit einem festen biokompatiblen partikelförmigen mineralischen, vorzugsweise pulverförmigen, Träger, der gezielt die angestrebten Tumorantigene adsorbieren kann und sie *in vivo* als Stoffe vektorisieren kann, die auf das Immunsystem wirken,
c. dafür sorgen, dass der partikelförmige mineralische Träger gezielt die interessierenden Tumorantigene adsorbiert,
d. Trennen des mineralischen Trägers vom nicht adsorbierten Tumorextrakt,
e. Gewinnen der interessierenden Tumorantigene in gereinigter Form und adsorbiert an dem partikelförmigen mineralischen Träger.

2. Verfahren nach Anspruch 1, **dadurch gekennzeichnet, dass** das Tumorextrakt, das in Schritt a. verwendet wird, ein autologes Tumorextrakt ist.

3. Verfahren nach Anspruch 1 oder 2, **dadurch gekennzeichnet, dass** es einen zusätzlichen Schritt des Vermischens der gereinigten und an dem Träger adsorbierten Tumorantigene mit mindestens einem Cofaktor, der die Wirkung der Tumorantigene auf das Immunsystem fördern kann, umfasst, wobei der Cofaktor vorzugsweise aus Tumorextrakten stammt, insbesondere aus zerkleinertem Material, Lyophilisaten, Dialysaten oder aus Zentrifugenbodensatz.

4. Verfahren nach einem der vorhergehenden Ansprüche, **dadurch gekennzeichnet, dass** es folgende Schritte umfasst:
a. Herstellen des Tumorextrakts, sodass die Suspendierung oder Löslichmachung der interessierenden Tumorantigene möglich ist,
b. Perkolieren der Suspension oder der Lösung durch mindestens eine Säule, die den partikelförmigen mineralischen Träger enthält,
c. Waschen der Säule mit Pufferlösungen mit einer bestimmten Ionenstärke und einem bestimmten pH-Wert,
d. Gewinnen des Trägers, der die Tumorantigene adsorbiert hat.

5. Verfahren nach Anspruch 4, **dadurch gekennzeichnet, dass** eine erste Säulenwäsche mit einem Phosphatpuffer oder einer NaCl-Lösung mit einer Konzentration von geringer als oder gleich 200 mM erfolgt und/oder dass eine zweite Wäsche mit einem Phosphatpuffer oder einer NaCl-Lösung mit einer Konzentration zwischen 300 und 500 mM erfolgt.

6. Verfahren nach einem der Ansprüche 4 oder 5, **dadurch gekennzeichnet, dass** die Herstellung des Tumorextrakts folgende Schritte umfasst:
a. eventuell Einfrieren des Tumorgewebes,
b. Zerkleinern des Tumorgewebes,
c. Löslichmachen oder Suspendieren der Tumorantigene in einer NaHCO₃-Lösung,
d. Zentrifugieren,
e. Trennen von Bodensatz und Überstand.

7. Verfahren nach einem der vorhergehenden Ansprüche, **dadurch gekennzeichnet, dass** der partikelförmige mineralische Träger aus Materialien ausgewählt wird, die alle oder einen Teil der folgenden Eigenschaften aufweisen:
- Vorhandensein von mindestens einer der ionisierten oder ionisierbaren Gruppen PO₄²⁻, OH⁻ und/oder Ca²⁺ an der Oberfläche des Materials,
- basischer pH-Wert der Oberfläche,
- negative Ladung der Oberfläche,
- hydrophob.

8. Verfahren nach einem der vorhergehenden Ansprüche, **dadurch gekennzeichnet, dass** der partikelförmige mineralische Träger ausgewählt wird:
- aus der Gruppe, die Calciumminerale umfasst, vorzugsweise Calciumphosphate und noch bevorzugter Hydroxyapatite,
oder
- aus Keramik, vorzugsweise einer Calciumphosphatkeramik, insbesondere einem Hydroxyapatit, einem Tri-, Tetra- oder Octacalciumphosphat in Partikelform.

9. Verfahren nach einem der vorhergehenden Ansprüche, **dadurch gekennzeichnet, dass** die Tumorantigene Folgendes umfassen:
a. Antigene, deren Expression in verschiedenen histologischen Krebsarten geteilt wird, und insbesondere die Antigene MAGE (melanoma antigens), BAGE (bladder antigens), GAGE (gastric antigens), RAGE (renal antigens), α-Fetoprotein, MUC1, HER-2/neu und ihre Gemische,
b. Antigene, die durch mutierte Gene codiert werden, und insbesondere die ras-Onkogene, Suppressorgene p53, β-Catenin, Cdk4, die Fusion der Antigene bcr/abl, Produkte viraler Gene (Epstein Barr, Hepatitis B, Papilloma), CASP-8 und ihre Gemische,
c. die tumorassoziierten Antigene karzinoembryonales Antigen, prostataspezifisches Antigen, RU2, Alt-M-CSF, Tyrosinase, Melan-A/MART1, Gp100, Gp75, Glioma associated oncogen, GLIP1, und/oder
d. Hitzeschockproteine (HSP, Heat Shock Protein), vorzugsweise das Protein gp96 und zugehörige Peptide.

10. Verfahren nach einem der vorhergehenden Ansprüche, **dadurch gekennzeichnet, dass** nach der Reinigung und Fixierung auf dem Träger nach Definition in Anspruch 7 oder 8 die Tumorantigene in einer NaCl-Lösung oder einem Phosphatbuffer 500 mM desorbiert werden, die anschließend auf zwischen 100 und 200 mM verdünnt und erneut über eine Säule geleitet wird, die einen Träger nach Definition in Anspruch 7 oder 8 mit einer Korngröße von unter 10 µm umfasst.
